# EUROPEAN PATENT APPLICATION

(11) **EP 0 661 316 A1**
(43) Date of publication of application: **05.07.1995**
(21) Application number: 94120101.4
(22) Date of filing: 19.12.1994
(51) Int. Cl.: C08G 18/32, C08G 18/38, C07C 275/10

(54) **Reactive urea/urethane compounds**

(30) Priority: 28.12.1993 US 174639
(71) Applicant: BASF CORPORATION, Mount Olive, New Jersey 08728-1234 (US)
(72) Inventor: Dantiki, Sudhar, Toledo, Ohio 43617 (US); Colyer, Emmerson Keith, Waterville, Ohio 43566 (US); Tye, Anthony J., Waterville, Ohio 43566 (US)
(74) Representative: Münch, Volker, Dr.

(57) **Abstract**

Novel reactive urea/urethane oligomers are disclosed. These oligomers have been found especially useful in coatings designed for high solids, high performance coatings. The coatings are especially useful as refinish automotive paint compositions.

## Description

### FIELD OF THE INVENTION

The present invention generally relates to novel reactive urea/urethane compounds, and to their preparation and use. The novel urea/urethane compounds comprise the reaction product of a polyisocyanate with preferably an alkanolamine forming, thereby, a reactive urea/urethane compound. These reactive urea/urethane compounds have been found especially useful as additives in paint compositions especially refinish paint compositions.

### BACKGROUND OF THE INVENTION

High solids coatings systems are known in the art and utilize various copolymers as the binder resins. A useful high solids coating system is one that is comprised of a polyhydroxy functional component together with a polyisocyanate component. Urethane polyols may be used as coreactants in such coating systems.

Several US and European patents deal with the process for the production of urethane adducts for use in coatings. For example, US 4,207,128 relates to a process for producing microporous coatings comprising a mixture of an NCO-prepolymer prepared from polyisocyanates with compounds containing at least two isocyanate reactive hydrogen atoms and about 3-40% by weight based on the NCO-prepolymer of a substantially inert, liquid organic compound.

US 4,235,766 teaches a coating composition having a prolonged pot life based on an organic polyhydroxy compound and an organic polyisocyanate.

US 4,096,291 discloses a melt coating composition for a heat resistant substrate with solvent free coating substances based on a combination of blocked polyisocyanates with compounds which contain hydrogen atoms reactive with NCO groups. Preferably the blocked isocyanate consist of at least 40 equivalents percent alkanol or cycloalkanol blocked polyisocyanates.

US patent 4,822,684 discloses a method for preparing multilayered coating comprising of an aqueous polyurethane dispersion and a polyamine. The isocyanate containing prepolymer was prepared from polyisocyanate and polyol or mixture of polyols having hydroxyl functionality of greater than two.

However, a review of the prior art indicates that it has been difficult to achieve sufficiently narrow molecular weight distributions in order to formulate high solids, high performance coating systems which possess both acceptable application characteristics, such as viscosity, leveling, appearance, and setting rate, and the resulting film properties. This is especially true for refinish auto paint where both requirements are critical to the final paint composition.

For example, U.S. Pat. Nos.4,485,228 and 4,540,766 describe high solids coating systems based, in part, upon relatively low molecular weight polyesterurethane polyols and crosslinking agents thereof. More particularly, U.S. Pat. No. 4,485,228 describes a two-pack system with a polyisocyanate crosslinker, while U.S. Pat. No. 4,540,766 describes a one-pack system with an aminoplast or blocked isocyanate crosslinker. The polyesterurethane polyols of these references are produced via the reaction of a polyisocyanate with a stoichiometric excess of a polyester polyol.

In related U.S. Pat. Nos. 4,540,771 and 4,605,724, the polyester polyols utilized for the polyurethane polyols are produced by reacting a polycarboxylic acid or lactone with a large excess of a low molecular weight polyol wherein, after completion of the reaction, the excess polyol is removed e.g. by distillation.

U.S. Pat. No. 4,548,998, like those references just mentioned, describes a high solids coating system based upon a polyesterurethane polyol, except that the polyesterurethane polyol is produced by isocyanate-free reaction of a polyester polyol, urea and a polyamine.

U.S. Pat. Nos. 4,524,192, 4,530,976, 4,533,703, 4,533,704 and EP-A-0139513 describe similar high solids coating systems which are based, in part, upon urethane-modified polyester polyols and crosslinking agents thereof. The urethane-modified polyester polyols are produced by reacting a urethane-modified diol component (from a diol and diisocyanate) with a diacid component and a second polyol.

In all of the aforementioned systems, molecular weight control of the polyesterurethane and urethane-modified polyester polyols is difficult because unwanted chain extension beyond the intended low molecular weight often results, leading to undesirably broad molecular weight distributions and high degrees of dispersion. The consequence, as indicated above for the acrylic and polyester based coatings, is that it is difficult to formulate high solids, high performance coating systems which possess both acceptable application and resulting film properties.

The problem of molecular weight control is somewhat alleviated by the process taught in U.S. Pat. No. 4,543,405. More particularly, this reference describes high performance, high solids coatings based upon low molecular weight polyurethane polyols and/or higher molecular weight prepolymers thereof, which polyurethane polyols are produced by reacting a polyisocyanate with a large excess of a polyol. After completion of the reaction, the excess polyol is removed, e.g. by distillation. Also relevant in this aspect is U.S. Pat. No. 4,288,577.

An obvious disadvantage of this procedure (and that of aforementioned U.S. Pat. Nos. 4,540,771 and 4,605,724) is that the distillation off of the excess polyol is inconvenient. Further, even at a high stoichiometric excess of the polyol reactant, unwanted chain extension beyond the intended low molecular weight may still occur. We have now found a novel class of urea/urethane compounds that overcome the disadvantages of the prior art and that are easily produced.

### SUMMARY OF THE INVENTION

The present invention relates to novel hydroxyl functional urea/urethane compounds, to their preparation and their use. In particular, the present invention relates to a process for the production of low molecular weight compounds containing reactive groups and to the reactive compounds produced thereby. Primary hydroxyl groups are especially useful reactive groups according to the present invention. Typically the preferred compounds are referred to as reactive compounds because they contain reactive hydroxyl groups. These reactive compounds of the present invention are essentially free of isocyanate groups, are of low to medium molecular weight, and contain free reactive groups other than isocyanates. These reactive groups are especially useful to provide for cross linking in coatings. Reactive oligomers so produced are especially useful in coating compositions and in particular in auto refinish paint compositions where these oligomers can be used as an additive to the coating composition and to accelerate the cross-linking in refinish paint compositions.

The reactive urea/urethane compounds, typically combine the reaction product of a polyisocyanate with an alkanolamine and optionally another hydroxyl functional reagent. The alkanolamine can be represented as: 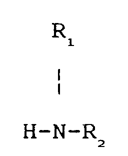
where
- R₁ =: alkyl or cycloaliphatic or benzylic type aryl (shielded aromatic ring) radical with a hydroxyl group.
- R₂ =: Hydrogen or alkyl or cycloaliphatic or benzylic type aryl (shielded aromatic ring) radical with or without a hydroxyl group.

The reactive urea/urethane compounds preferably contain primary hydroxyl groups ,low molecular weight, preferably with a weight average molecular weight less than 6000, as measured by gel permeation chromatography relative to polystyrene standards, a low polydispersity, preferably less than 2.0, and a high degree of crystallinity.

Aliphatic, aromatic and/or other groups can also be incorporated into the urea/urethane compound by the reaction of -N=C=O groups on the polyisocyanate with other reactants containing abstractable hydrogens such as alcohols.

### DETAILED DESCRIPTION OF THE INVENTION

The present invention relates to novel hydroxyl functional urethane/urea compounds, their preparation and use. In particular, the present invention relates to a process for the production of compounds containing reactive groups and to the reactive compounds produced thereby. Primary hydroxyl groups are especially useful reactive groups and are especially useful on polymers for use in refinish paint compositions. The preferred urea/urethane compounds are referred to as reactive urea/urethane compounds because they contain reactive groups such as the preferred hydroxyl groups. Reactive groups such as carboxyl, epoxy, mercaptan, alkoxysilane, etc. may also be useful on urea/urethane compounds for use in refinish paint compositions.

The present invention also relates to a process for the production of urea/urethane compounds containing reactive groups such as the preferred primary hydroxyl groups. The products are referred to as reactive urea/urethane compounds because they contain reactive groups such as hydroxyl groups which can be used to cross link with other compounds containing isocyanates or other compounds that contain groups that can cross-link with hydroxyl groups such as a melamine-formaldehyde resin. An oligomer is a low molecular weight polymer with a degree of polymerization less than ten.

The reactive oligomers of the present invention are especially useful in automotive refinish coating compositions. These coatings can be designed as air-dry, oven bake or radiation cured coatings. The coating compositions wherein these reactive urethane oligomers can be used as covehicles in single stage topcoat formulation as well as the clearcoat and base coat formulations are described in related patent applications S.N. , S.N. & S.N. . Preferred compositions where the oligomers can be used as covehicles in paint compositions are described in these related copending applications.

The coating compositions of the present invention typically comprise the following:
(i) a principal resin such as an acrylic, polyester, acrylester or urethane resin containing functional groups such as hydroxyl, carboxyl, and amino functional groups.
(ii) a reactive urea/urethane compound compatible with the principal resin.
(iii) a curing agent such as an isocyanate and/or a mixture of curing agents, and optionally
(iv) additives such as accelerators, anti-sagging agents, flow agents, driers, UV absorbers etc. known in the coating art.

Suitable amines are the alkanolamines and include but are not limited to: 2-(propylamino)ethanol, 2-(Methylamino)ethanol, 2-(Ethylamino)ethanol, 2-(t-Butylamino)ethanol, 2-(n-Butylamino)ethanol, Diisopropylamine and Diethanolamine.

Suitable polyisocyanates useful in the invention include isophorone diisocyanate, tetramethyl xylene diisocyanate, dicyclohexylmethane-4,4'-diisocyanate, the isocyanurate of isophorone diisocyanate and the isocyanurate of hexamethylenediisocyanate.

In addition, aliphatic, aromatic and/or other groups can also be incorporated into the urea/urethane compound by the reaction of -N=C=O groups on the polyisocyanate with other reactants containing abstractable hydrogens such as alcohols.

The reactive oligomers typically combine the reaction product of an isocyanate with an alkanolamine that can be represented as: 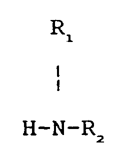
where
- R₁ =: alkyl or cycloaliphatic or benzylic type aryl (shielded aromatic ring) radical with a hydroxyl group.
- R₂ =: Hydrogen or alkyl or cycloaliphatic or benzylic type aryl (shielded aromatic ring) radical with or without a hydroxyl group.

The reactive oligomers preferably contain primary hydroxyl groups , low molecular weight, preferably with a weight average molecular weight less than 6000, as measured by gel permeation chromatography relative to polystyrene standards, a low polydispersity , preferably less than 2.0, and a high degree of crystallinity.

Aliphatic, aromatic and/or other groups can also be incorporated into the urea/urethane compound by the reaction of -N=C=O groups on the polyisocyanate with other reactants containing abstractable hydrogens such as alcohols.

The following examples are intended to illustrate the invention:

### EXAMPLE 1

This example of the invention utilized two reactions including one to make an intermediate.
I. Intermediate
1. Composition:

| | | |
|---|---|---|
| A. | Vestanat T-1890 E | 738.6 (Huls-IPDI isocyanurate, 70% NV, 12% -N=C=O) |
| | n-Butyl acetate | 175.6 (urethane grade) |
| | Octadecanol | 80.3 |
| | Triisodecylphosphite | 1.0 |
| B. | Dibutyltin dilaurate | 0.0056 |
| | n-Butyl acetate | 4.5 (urethane grade) |

2. Process:
A. Add components "A" to reactor and heat to 65-75 C.
B. At 65-75 C, add components "B" and hold until the %NCO becomes constant.
C. Cool and store Intermediate under Nitrogen until needed.

II. Finished Urethane/Urea Compound
1. Composition:

| | | |
|---|---|---|
| A. | 2-(t-Butylamino)ethanol | 146.2 |
| | n-Butyl acetate | 126.5 (urethane grade) |
| B. | Intermediate | 723.0 (from I) |
| C. | n-Butyl acetate | 4.3 (urethane grade) |

2. Process:
A. Add component "A" to reactor and heat to 50C.
B. Add component "B" to reactor over 180 minutes.

- R₂ =: Hydrogen or alkyl or cycloaliphatic or benzylic type aryl (shielded aromatic ring) radical with or without a hydroxyl group.

2. A reactive urea/urethane compound according to claim 1 wherein the oligomer has a weight average molecular weight less than 6000, as measured by gel permeation chromatography relative to polystyrene standards, and a polydispersity of less than 2.0.
3. A reactive urea/urethane comprised of the reaction product of a polyisocyanate and a mixture of a hydroxyl functional component with structure : 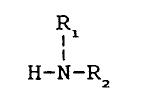 where
   - R₁ =: alkyl or cycloaliphatic or benzylic type aryl (shielded aromatic ring) radical with a hydroxyl group.
   - R₂ =: Hydrogen or alkyl or cycloaliphatic or benzylic type aryl (shielded aromatic ring) radical with or without a hydroxyl group.
   and a monofunctional hydroxyl component.
4. A reactive urea/urethane compound according to claim 3 wherein the oligomer has a weight average molecular weight less than 6000, as measured by gel permeation chromatography relative to polystyrene standards, and a polydispersity of less than 2.0.
5. A reactive urea/urethane compound according to claims 1-4 where the polyisocyanate is a diisocyanate.
6. A reactive urea/urethane compound according to claims 1-4 where the polyisocyanate is an isocyanate functional isocyanurate trimer.

C. Flush with component "C" then hold at temperature until % NCO = 0 ( ASTM D2572-87).

### EXAMPLE 2

1. Composition:

| | | |
|---|---|---|
| A. | 2-(Methylamino)ethanol | 101.8 |
| | n-Butyl acetate | 209.5 |
| | Triisodecylphosphite | 2.0 |
| | Dibutyltin dilaurate | 0.005 |
| B. | Vestanat T-1890E | 499.7 |
| C. | n-Butyl acetate | 31.3 |
| D. | -Caprolactone | 146.3 |
| | Stannous octoate | 1.46 |
| E. | n-Butyl acetate | 7.9 |

2. Process:
A. With components "A" in the reactor, heat to 50 C.
B. Add component "B" to the reactor over 180 minutes. Flush with component "C".
C. Hold at temperature (50°) until the % NCO = 0.
D. When the % NCO = 0 ( ASTM D2572-87 ), add components "D" to the reactor. Flush with component "E".
E. Heat to reflux and hold at reflux for 240 minutes.

## Claims

1. A reactive urea/urethane compound comprised of the reaction product of a polyisocyanate and a hydroxyl functional component with structure : 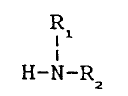 where
R₁ = alkyl or cycloaliphatic or benzylic type aryl (shielded aromatic ring) radical with a hydroxyl group.
